# EUROPEAN PATENT APPLICATION

(11) **EP 4 339 280 A1**
(43) Date of publication of application: **20.03.2024**
(21) Application number: 22807607.1
(22) Date of filing: 28.03.2022
(51) Int. Cl.: C12N 5/077, C12N 5/071

(54) **METHOD FOR PRODUCING HEART ORGANOID DERIVED FROM HUMAN PLURIPOTENT STEM CELL AND HEART ORGANOID DERIVED FROM HUMAN PLURIPOTENT STEM CELL PRODUCED THEREBY**

(30) Priority: 10.05.2021 KR 20210060265
(71) Applicant: NEXEL CO., LTD., Seoul 02580 (KR)
(72) Inventor: WOO, Donghun, Seoul 07900 (KR); KIM, Hyeji, Seoul 07917 (KR); KIM, Mijin, Seoul 03472 (KR); LEE, Hanbyeol, Incheon 22389 (KR); IM, Jeongsuk, Siheung-si Gyeonggi-do 14905 (KR)
(74) Representative: dompatent von Kreisler Selting Werner - Partnerschaft von Patent- und Rechtsanwälten mbB
(86) International application number: PCT/KR2022/004329
(87) International publication number: WO 2022/239959

(57) **Abstract**

The present invention relates to a method for producing human pluripotent stem cell-derived cardiac organoids, the method comprising steps of: (A) preparing embryonic bodies having a diameter of 100 um to 130 um by culturing human pluripotent stem cells (hPSCs); (B) culturing the embryonic bodies, prepared in step (A), until the embryonic bodies have a diameter of 200 um to 250 um; (C) differentiating the human pluripotent stem cell-derived embryonic bodies having a diameter of 200 um to 250 µm, obtained in step (B), into structures comprising mesoderm cells and endoderm cells; (D) differentiating the structures comprising mesoderm cells and endoderm cells, obtained in step (C), into cardiac organoids comprising self-organized cardiomyocytes, fibroblasts and vascular endothelial cells; and (E) culturing and maturing the cardiac organoids obtained by differentiation in step (D), wherein the cardiac organoids matured in step (E) contain self-organized cardiomyocytes, fibroblasts and vascular endothelial cells at a cell number ratio of 55 to 65: 15 to 30: 15.

## Description

### Technical Field

The present invention relates to a method for producing human pluripotent stem cell-derived cardiac organoids and human pluripotent stem cell-derived cardiac organoids produced thereby.

### Background Art

Until recently, a method for differentiating human pluripotent stem cells (hPSCs) from most cells constituting the human body has been developed due to the continuous development of technolog. However, the cells differentiated by two-dimensional culture have lower functionality than actual human cells due to their considerably low maturity level, and thus have clear limitations in their use for drug toxicity evaluation, drug screening, or cell therapy.

Accordingly, studies on various technologies related to cell differentiation and culture have been conducted to produce cells which have a maturity level comparable to that of actual human cells and have sufficient functionality. In particular, organoids, which may exhibit sufficient functionality and mimic actual organs in which cells do not exist individually but exist in clusters, have been researched and developed worldwide.

Organoids that can mimic the structure and function of real human organs are possible through continuous cell division and differentiation from organ stem cells, such as intestine and brain, which are known to have organ-specific stem cells. However, In the case of a heart in which organ-specific stem cells do not exist, this method may not be applied, and a method of producing cardiac organoids by separately differentiating various cells constituting the heart and then mixing the cells has been suggested as the only method.

In this regard, prior art documents related to the production of cardiac organoids include US Patent Publication No. 2020-0283735, entitled "Organoid and method for producing the same" (hereinafter referred to as "conventional art").

However, conventional methods for producing cardiac organoids, including the conventional art, are based on simply mixing differentiated cardiac constituent cells or performing culturing depending on an extracellular matrix composed of specific components, and overlook the fact that the characteristics of development of various cardiac constituent cells, which interact with one another from the initial stage of development, affect the function of the heart.

As a result, when various cells constituting the heart fail to self-organize through interaction from the initial stage of development while differentiating, a problem arises in that the resulting cardiac organoids are insufficient to mimic the structure and function of an actual heart, and thus the use thereof in drug screening and cardiac toxicity evaluation through disease modeling during new drug development is quite insufficient to derive significant results.

### DISCLOSURE

### Technical Problem

The present invention has been made in order to solve the above-described problem, and an object of the present invention is provide a method for producing cardiac organoids which sufficient to mimic the structure and function of an actual heart, including simultaneous differentiation into various cardiac constituent cells from human pluripotent stem cells and the self-organization of cells through interaction from the initial stage of development, and are sufficient to mimic the structure and function of an actual heart.

### Technical Solution

To achieve the above object, a method for producing human pluripotent stem cell-derived cardiac organoids according to the present invention comprises: step (A) of preparing embryonic bodies having a diameter of 100 um to 130 um by culturing human pluripotent stem cells (hPSCs); step (B) of culturing the embryonic bodies, prepared in step (A), until the embryonic bodies have a diameter of 200 um to 250 µm; step (C) of differentiating the human pluripotent stem cell-derived embryonic bodies having a diameter of 200um to 250pm, obtained in step (B), into structures comprising mesoderm cells and endoderm cells; step (D) of differentiating the structures comprising mesoderm cells and endoderm cells, obtained in step (C), into cardiac organoids comprising self-organized cardiomyocytes, fibroblasts and endothelial cells; and step (E) of culturing and maturing the cardiac organoids obtained by differentiation in step (D), wherein the cardiac organoids matured in step (E) comprise self-organized cardiomyocytes, fibroblasts and endothelial cells at a cell number ratio of 55 to 65: 15 to 30: 15.

In the method, the human pluripotent stem cells in step (A) are at least one cell type selected from among human embryonic stem cells (hESCs) and human induced pluripotent stem cells (hiPSCs).

In addition, step (A) is a step of preparing embryonic bodies having a diameter of 100 um to 130 µm by plating human pluripotent stem cells in an mTeSR1 medium supplemented with a Rho-associated kinase (ROCK) inhibitor (Y-27632) and culturing the cells for 1 day.

Furthermore, step (B) is a step of preparing human pluripotent stem cell-derived embryonic bodies having a diameter of 200 um to 250 um by culturing the embryonic bodies having a diameter of 100 um to 130 um, prepared in step (A), in mTeSR1 medium free of a Rho-associated kinase (ROCK) inhibitor (Y-27632) for 2 to 3 days.

Step (C) comprises: step (C-1) of culturing the human pluripotent stem cell-derived embryonic bodies having a diameter of 200 um to 250 um, prepared in step (B), for 2 days in an RPMI 1640 medium supplemented with B-27 supplement (minus insulin), CHIR99021, BMP4 (bone morphogenetic protein-4) and Activin A; and step (C-2) of differentiating the human pluripotent stem cell-derived embryonic bodies, cultured in step (C-1), into structures comprising mesoderm cells and endoderm cells by culturing the embryonic bodies for 2 days in an RPMI 1640 medium supplemented with B-27 supplement (minus insulin), XAV939 and L-ascorbic acid.

Here, step (C-1) is a step of culturing the human pluripotent stem cell-derived embryonic bodies having a diameter of 200 um to 250 um for 2 days in an RPMI 1640 supplemented with B-27 supplement (minus insulin), 6 µM to 8 µM of CHIR99021 as a GSK-3β (glycogen synthase kinase-3β) inhibitor for Wnt signal activation, 9 ng/ml to 10 ng/ml of BMP4 as a component for BMP signal activation, and 8 ng/ml to 10 ng/ml of Activin A as a component for TGF-β (transforming growth factor-β) signal activation.

In addition, step (C-2) is a step of culturing the human pluripotent stem cell-derived embryonic bodies, cultured in step (C-1), for 2 days in an RPMI 1640 medium supplemented with B-27 supplement (minus insulin), 8 µM to 12 µM of XAV939 as a component for Wnt signaling inhibition and 40 ug/ml to 60 µg/ml of L-ascorbic acid as a component for BMP signal activation.

Moreover, step (D) comprises: step (D-1) of inducing differentiation into cardiomyocytes by culturing the structures comprising mesoderm cells and endoderm cells, obtained in step (C), for 2 days in a RPMI 1640 medium supplemented with B-27 supplement (minus insulin) and 40 ug/ml to 60 µg/ml of L-ascorbic acid; step (D-2) of inducing differentiation into fibroblasts and endothelial cells by culturing the structures, whose differentiation into cardiomyocytes has been induced in step (D-1), for 2 days in the RPMI 1640 medium used in step (D-1) and further supplemented with 20 ng/ml to 40 ng/ml of BMP4, 20 ng/ml to 40 ng/ml of VEGF (vascular endothelial growth factor) and 20 ng/ml to 40 ng/ml of FGF2 (fibroblast growth factor 2); step (D-3) of culturing the structures, whose differentiation into cardiomyocytes, fibroblasts and endothelial cells has been induced in step (D-2), for 2 days in an RPMI 1640 medium supplemented with B-27 supplement (minus vitamin A), 40 µg/ml to 60 µg/ml of L-ascorbic acid, 20 ng/ml to 40 ng/ml of BMP4, 20 ng/ml to 40 ng/ml of VEGF (vascular endothelial growth factor) and 20 ng/ml to 40 ng/ml of FGF2 (fibroblast growth factor 2); and step (D-4) of obtaining cardiac organoids through differentiation and self-organization by culturing the structures, cultured in step (D-3), for 2 days in an RPMI 1640 medium supplemented with B-27 supplement (minus vitamin A), 20 ng/ml to 40 ng/ml of BMP4, 20 ng/ml to 40 ng/ml of VEGF (vascular endothelial growth factor) and 20 ng/ml to 40 ng/ml of FGF2 (fibroblast growth factor 2).

In addition, step (E) is a step of maturing the cardiac organoids comprising self-organized cardiomyocytes, fibroblasts and endothelial cells, obtained through differentiation in step (D-4), by culturing the cardiac organoids for 10 to 20 days in an RPMI 1640 medium supplemented with B-27 supplement (minus vitamin A), 20 ng/ml to 40 ng/ml of VEGF (vascular endothelial growth factor), 20 ng/ml to 40 ng/ml of FGF2 (fibroblast growth factor 2), and 10 ng/ml to 15 ng/ml of SB431542 as a component for TGF-β (transforming growth factor-β) signal inhibition.

Meanwhile, to achieve the above object, the human pluripotent stem cell-derived cardiac organoids according to the present invention are produced through the above-described method for producing human pluripotent stem cell-derived cardiac organoids.

### Advantageous Effects

The present invention has the following effects.

First, overcoming the limitations of the absence of cardiac stem cells, cardiomyocytes, cardiac fibroblasts and endothelial cells constituting heart are simultaneously differentiated from human pluripotent stem cells, and self-organization is achieved through interaction from the early stage of development to differentiate in culture. Thus, cardiac organoids with conditions sufficient to mimic the structure and function of an actual heart can be prepared.

Second, it is possible to dramatically increase the efficiency of new drug development by using the cardiac organoids, which are cardiac organoids in a state sufficient to mimic the structure and function of the heart can be used for drug screening and cardiotoxicity evaluation through disease modeling during new drug development which can dramatically increase the efficiency of new drug development.

Third, the present invention may contribute to technological development in the field of disease research by using the human pluripotent stem cell-derived cardiac organoids can be used as an alternative model for studying the mechanism of heart-related disease , contributing to the advancement of technology in the field of disease research. Human pluripotent stem cell-derived cardiac organoids can be used as an alternative model for studying the mechanism of heart-related diseases, contributing to the advancement of technology in the field of disease research.

### Brief Description of Drawings

FIG. 1 is a flow chart showing a method for producing human pluripotent stem cell-derived cardiac organoids according to the present invention.
FIGS. 2 and 3 are photographs showing the results of observing changes in the size and cell number of embryoid bodies depending on an embryoid body preparation step and a culturing step in a method for producing human pluripotent stem cell-derived cardiac organoids according to the present invention.
FIG. 4 depicts micrographs showing time-dependent states during the process of differentiation from human pluripotent stem cells through embryoid bodies into cardiac organoids in the method for producing human pluripotent stem cell-derived cardiac organoids according to the present invention.
FIG. 5 is a graph showing time-dependent changes in diameter during the process of differentiation from human pluripotent stem cells through embryoid bodies into cardiac organoids in the method for producing human pluripotent stem cell-derived cardiac organoids according to the present invention.
FIG. 6 is a graph showing time-dependent changes in the percentage of beating during the process of differentiation from human pluripotent stem cells through embryoid bodies into cardiac organoids in the method for producing human pluripotent stem cell-derived cardiac organoids according to the present invention.
FIG. 7 shows fluorescence staining images of human pluripotent stem cell-derived cardiac organoids produced through the method for producing human pluripotent stem cell-derived cardiac organoids according to the present invention.
FIG. 8 is a schematic diagram summarizing culture conditions and periods for each of experimental groups in the method for producing human pluripotent stem cell-derived cardiac organoids according to the present invention.
FIGS. 9 and 10 are graphs showing flow cytometry results for each experimental group of human pluripotent stem cell-derived cardiac organoids produced through the method for producing human pluripotent stem cell-derived cardiac organoids according to the present invention.
FIGS. 11 to 13 are graphs showing the results of measuring gene expression in each experimental group of human pluripotent stem cell-derived cardiac organoids produced through the method for producing human pluripotent stem cell-derived cardiac organoids according to the present invention.
FIGS. 14 and 15 show fluorescence staining images of each experimental group of human pluripotent stem cell-derived cardiac organoids produced through the method for producing human pluripotent stem cell-derived cardiac organoids according to the present invention.
FIGS. 16 and 17 depict photographs and a graph, which show the results of calcium imaging of each experimental group of human pluripotent stem cell-derived cardiac organoids produced through the method for producing human pluripotent stem cell-derived cardiac organoids according to the present invention.

### Mode for Invention

Preferred embodiments of the present invention will be described in more detail with reference to the accompanying drawings. In the following description, the description of already known technical features will be omitted or compressed for the sake of brevity of description.

### 1. Description of method for producing human pluripotent stem cell-derived cardiac organoids

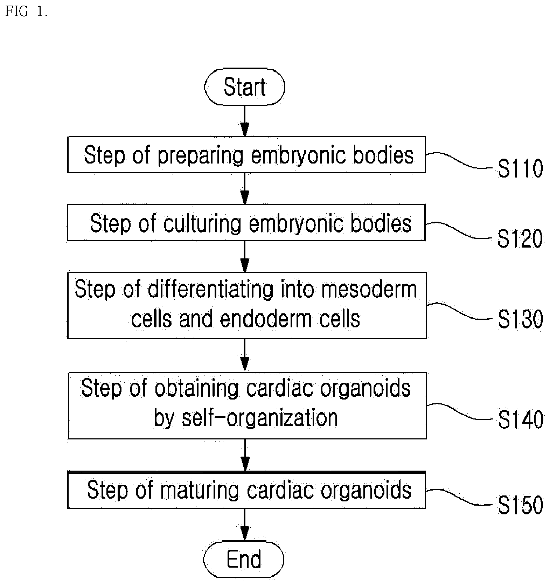

Hereinafter, each step of the method for producing human pluripotent stem cell-derived cardiac organoids according to the present invention will be described in detail with reference to FIG. 1.

### (1) Step of preparing embryonic bodies <S110, step A>

In this step (S110), a process of preparing embryonic bodies having a diameter of 100 um to 130 um by culturing human pluripotent stem cells (hPSCs) is performed.

As used herein, the term "human pluripotent stem cells (hPSCs)" refers to hPSCs themselves, but in a more specific sense, may refer to at least one cell type selected from among human embryonic stem cells (hESCs) and human induced pluripotent stem cells (hiPSCs).

Specifically, in step (S110) of preparing embryonic bodies, human pluripotent stem cells in culture are harvested and then seeded in a 6-well plate at a density of 1.5×10⁶ cells/well using an mTeSR1 medium (Stem Cell Technologies, Canada) supplemented with 10 µM of a Rho-associated kinase) inhibitor (ROCK) (Y-27632, Tocris Bioscience, UK).

Here, the 6-well plate, in which the human pluripotent stem cells are plated, is not coated for prevent cell adhesion, and Matrigel that is generally used for organoid culture is also not used. In addition, a low-attachment plate may be used instead of the 6-well plate.

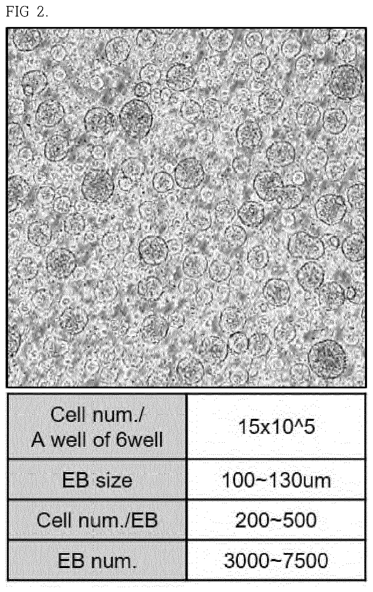

As shown in Figure 2, after one day of culturing, embryonic bodies, each composed of about 200 to 500 cells and having a diameter of 100 um to 130 um, are prepared in each well.

Thus, when 1.5 × 10⁶ human pluripotent stem cells are plated in each well of the 6-well plate, about 3,000 to 7,500 embryonic bodies having a diameter of 100 um to 130 um are produced after 1 day of culturing.

### (2) Step of culturing embryonic bodies <S120, step B>

In this step (S120), a process of further culturing the embryo bodies are prepared in the embryo body preparation step (S110) to a diameter of 200 µm to 250 µm is performed.

Specifically, step (S120) is an embryo body having a diameter of 100 µm to 130 µm prepared through the preparing embryonic body step (S110) in an mTeSR culture medium without ROCK (Rho-associated kinase) inhibitor (Y27632). There are cultured for 2 to 3 days to prepare a human pluripotent stem cell-derived embryo with a diameter of 200 µm to 250 µm.

Here, the culturing process using the mTeSR1 medium free of a Rho-associated kinase (ROCK) inhibitor (Y-27632) is preferably performed for 2 to 3 days while the medium is replaced with fresh medium one every day.

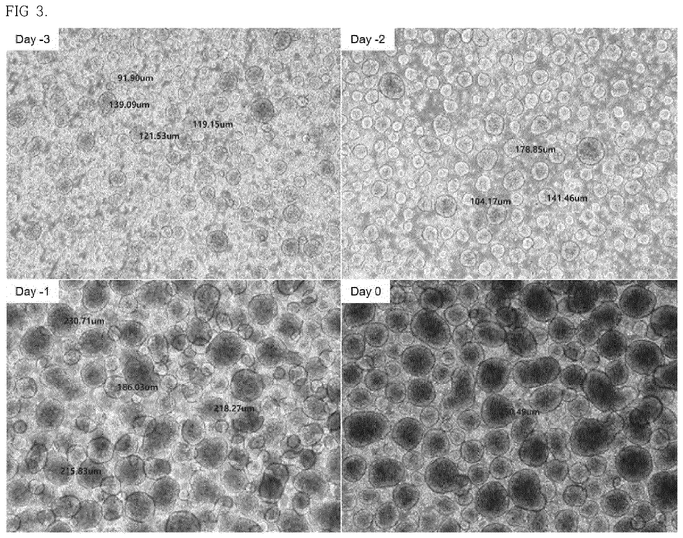

Accordingly, as shown in FIG. 3, on day 1 of culturing when embryo body preparation step (S110) is performed, embryo bodies having a diameter of 100 um to 130 um are prepared as shown in the photograph marked "Day-3", and after 2 to 3 days of additional culturing under varying medium conditions in embryonic body culture step (S120), the diameter increases gradually and comes within a diameter range of 250 um as shown in the photograph marked "Day 0".

### (3) Step of differentiating into mesoderm and endoderm cells <S130, step C>

In this step (S130), a process of differentiating the human pluripotent stem cell-derived embryonic bodies having a diameter of 200 um to 250 µm, obtained in embryo body culturing step (S120), into structures comprising mesoderm cells and endoderm cells is performed.

More specifically, this step (S130) of differentiating into mesoderm and endoderm cells is subdivided into 2 sequential steps. First, a first differentiation process (step (C-1)) of culturing the human pluripotent stem cell-derived embryonic bodies having a diameter of 200 um to 250 µm, prepared in embryonic body culturing step (B) for 2 days . The culture medium is RPMI 1640 supplemented with B-27 supplement (minus insulin), CHIR99021, BMP4 (bone morphogenetic protein-4) and activin A.

Specifically, the medium, in which first culturing of the human pluripotent stem cell-derived embryonic bodies having a diameter of 200 um to 250 µm, prepared in embryonic body culturing step (B), is performed for 2 days, is an RPMI 1640 supplemented with B-27 supplement (minus insulin), 6 µM to 8 µM of CHIR99021, 9 ng/ml to 10 ng/ml of BMP4, and 8 ng/ml to 10 ng/ml of Activin A.

Here, CHIR99021 added to the RPMI 1640 medium used in the first differentiation process in step (S130) of differentiating into mesoderm and endoderm cells is a GSK-3β (glycogen synthase kinase-3β) inhibitor for Wnt signal activation and is most preferably used at a concentration of 6 µM.

In addition, BMP-4 (bone morphogenetic protein-4) added to the RPMI 1640 medium used in the first differentiation process in step (S130) of differentiating into mesoderm and endoderm cells is a component for BMP signal activation, and is most preferably used at a concentration of 10 ng/ml.

Furthermore, Activin A added to the RPMI 1640 medium used in the first differentiation process in step (S130) of differentiating into mesoderm and endoderm cells is a component for TGF-β (transforming growth factor-β) signal activation, and is most preferably used at a concentration of 10 ng/ml.

The first differentiation process is followed by a second differentiation process (step C-2) of culturing the human pluripotent stem cell-derived embryonic bodies, cultured in the first differentiation process in step (130) of differentiating into mesoderm and endoderm cells, for 2 days in an RPMI 1640 medium supplemented with B-27 supplement (minus insulin), XAV939 and L-ascorbic acid, thus differentiating the embryonic bodies into structures comprising mesoderm cells and endoderm cells.

Specifically, the medium, in which second culturing of the human pluripotent stem cell-derived embryonic bodies, cultured in the first differentiation process in step (130) of differentiating into mesoderm and endoderm cells, is performed for 2 days, is an RPMI 1640 medium supplemented with B-27 supplement (minus insulin; Thermo Fisher Scientific, USA), 8 µM to 12 µM of XAV939 and 40 µg/ml to 60 µg/ml of L-ascorbic acid.

Here, XAV939 added to the RPMI 1640 medium used in the second differentiation process in step (130) of differentiating into mesoderm and endoderm cells is a component for Wnt signal inhibition and is most preferably used at a concentration of 10 µM.

In addition, XAV939 added to the RPMI 1640 medium used in the second differentiation process in step (130) of differentiating into mesoderm and endoderm cells is an inhibitor of Wnt production (IWP)-2, 3, 4 according to a practice, and IWP-2, 3, 4 may be used at the same concentration as described above.

In addition, L-ascorbic acid added to the RPMI 1640 medium used in the second differentiation process in step (S130) of differentiating into mesoderm and endoderm cells is a component for Wnt signal inhibition and is most preferably used at a concentration of 50 µg/ml.

### (4) Step of obtaining cardiac organoid self-organization <S140, step D>

In this step (S140), a process of differentiating the structures comprising mesoderm cells and endoderm cells, obtained in step (S130) of differentiating into mesoderm and endoderm cells, into cardiac organoids comprising self-organized cardiomyocytes, fibroblasts and endothelial cells, is performed.

The Cardiac organoid self-organization step S140 is sequentially performed over four steps. First, a process of culturing a composition containing mesoderm cells and endoderm cells differentiated through mesoderm and endoderm cell differentiation (S130) is carried out in the first stage of RPMI 1640 with B-27 supplement (minus insulin) and L-ascorbic acid (L-Ascorbic acid) with a concentration of 40 to 60 µg/ml.

Here, L-ascorbic acid added to the RPMI 1640 medium used in the first organization process in step (S140) of obtaining cardiac organoids through self-organization is most preferably used at a concentration of 50 µg/ml.

The first organization step is followed by a second organization process (step (D-2)) of inducing differentiation into fibroblasts and endothelial cells by culturing the structures, whose differentiation into cardiomyocytes has been induced in the first organization process (step (D-1)), for 2 days in the RPMI 1640 medium used in the first organization process (step (D-1)) and further supplemented with 20 ng/ml to 40 ng/ml of BMP4, 20 ng/ml to 40 ng/ml of VEGF (vascular endothelial growth factor) and 20 ng/ml to 40 ng/ml of FGF2 (fibroblast growth factor 2).

Here, BMP4 (bone morphogenetic protein-4) added to the RPMI 1640 medium used in the second organization process in step (S140) of obtaining cardiac organoids through self-organization is a component for BMP signal activation, and is most preferably used at a concentration of 30 ng/ml.

In addition, VEGF (vascular endothelial growth factor) added to the RPMI 1640 medium used in the second organization process in step (S140) of obtaining cardiac organoids through self-organization is a component involved in vascular differentiation and formation, and is preferably used at a concentration of 30 ng/ml.

Furthermore, FGF2 (fibroblast growth factor 2) added to the RPMI 1640 medium used in the second organization process in step (S140) of obtaining cardiac organoids through self-organization is a component for FGF signal activation, and is preferably used at a concentration of 30 ng/ml.

The second organization process is followed by a third organization process (step (D-3)) of culturing the structures, whose differentiation into cardiomyocytes, fibroblasts and endothelial cells has been induced in the second organization process (step (D-2)), for 2 days in an RPMI 1640 medium supplemented with B-27 supplement (minus vitamin A), 40 µg/ml to 60 µg/ml of L-ascorbic acid, 20 ng/ml to 40 ng/ml of BMP4, 20 ng/ml to 40 ng/ml of VEGF (vascular endothelial growth factor) and 20 ng/ml to 40 ng/ml of FGF2 (fibroblast growth factor 2).

Here, L-ascorbic acid added to the RPMI 1640 medium in the third organization process in step (S140) of obtaining cardiac organoids through self-organization is most preferably used at a concentration of 50 µg/ml.

In addition, BMP4 (bone morphogenetic protein-4) added to the RPMI 1640 medium in the third organization process in step (S140) of obtaining cardiac organoids through self-organization is a component for BMP signal activation, and is preferably used at a concentration of 30 ng/ml.

Furthermore, VEGF (vascular endothelial growth factor) added to the RPMI 1640 medium in the third organization process in step (S140) of obtaining cardiac organoids through self-organization is a component involved in vascular differentiation and formation, and is preferably used at a concentration of 30 ng/ml.

In addition, FGF2 (fibroblast growth factor 2) added to the RPMI 1640 medium in the third organization process in step (S140) of obtaining cardiac organoids through self-organization is a component for FGF signal activation, and is preferably used at a concentration of 30 ng/ml.

Finally, the third organization process is followed by a fourth organization process (step (D-4)) of obtaining cardiac organoids through differentiation and self-organization by culturing the structures, cultured in the third organization process (step (D-3)), for 2 days in an RPMI 1640 medium supplemented with B-27 supplement (minus vitamin A), 20 ng/ml to 40 ng/ml of BMP4, 20 ng/ml to 40 ng/ml of VEGF (vascular endothelial growth factor) and 20 ng/ml to 40 ng/ml of FGF2 (fibroblast growth factor 2).

Here, BMP4 (bone morphogenetic protein-4) added to the RPMI 1640 medium used in the fourth organization process in step (S140) of obtaining cardiac organoids through self-organization is a component for BMP signal activation, and is preferably used at a concentration of 30 ng/ml.

In addition, VEGF (vascular endothelial growth factor) added to the RPMI 1640 medium used in the fourth organization process in step (S140) of obtaining cardiac organoids through self-organization is a component involved in vascular differentiation and formation, and is preferably used at a concentration of 30 ng/ml.

Furthermore, FGF2 (fibroblast growth factor 2) added to the RPMI 1640 medium used in the fourth organization process in step (S140) of obtaining cardiac organoids through self-organization is a component for FGF signal activation, and is preferably used at a concentration of 30 ng/ml.

After step (S140) of obtaining cardiac organoids through self-organization is completed through these sequential processes, cardiac organoids comprising self-organized cardiomyocytes, fibroblasts and endothelial cells are obtained.

### (5) Step of maturing cardiac organoids <S150, step E>

In this step (S150), a process of culturing and maturing the cardiac organoids obtained by differentiation in step (S140) of obtaining cardiac organoids through self-organization is performed.

Specifically, in step (S150) of maturing cardiac organoids, the cardiac organoids obtained in step (S140) of obtaining cardiac organoids through self-organization are cultured and matured in a maturation medium for 10 to 20 days so that they comprise self-organized cardiomyocytes, fibroblasts and endothelial cells.

Here, the medium for maturation of the cardiac organoids is an RPMI 1640 medium supplemented with B-27 supplement (minus vitamin A), 20 ng/ml to 40 ng/ml of VEGF (vascular endothelial growth factor), 20 ng/ml to 40 ng/ml of FGF2 (fibroblast growth factor 2), and 10 ng/ml to 15 ng/ml of SB431542 as a component for TGF-β (transforming growth factor-β) signal inhibition.

In addition, VEGF (vascular endothelial growth factor) added to the RPMI 1640 medium used in step (S150) of maturing cardiac organoids is a component involved in vascular differentiation and formation, and is preferably used at a concentration of 30 ng/ml.

Furthermore, FGF2 (fibroblast growth factor 2) added to the RPMI 1640 medium used in step (S150) of maturing cardiac organoids is a component for FGF signal activation, and is preferably used at a concentration of 30 ng/ml.

In addition, SB431542 added to the RPMI 1640 medium used in step (S150) of maturing cardiac organoids is a component for TGF-β (transforming growth factor-β) signal inhibition, and is preferably used at a concentration of 10 ng/ml.

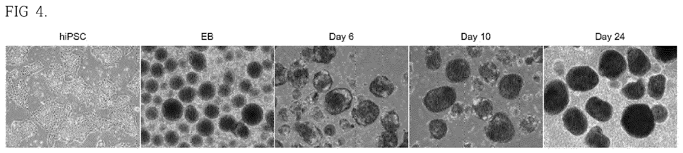

As a result, as shown in FIG. 4, it can be confirmed that embryonic bodies (EBs) are obtained from human induced pluripotent stem cells (hiPSCs), and then differentiate into mesoderm and endoderm cells on day 6, which then differentiate into cardiac organoids comprising self-organized cardiomyocytes, fibroblasts and endothelial cells on day 10, and matured cardiac organoids are obtained on day 24.

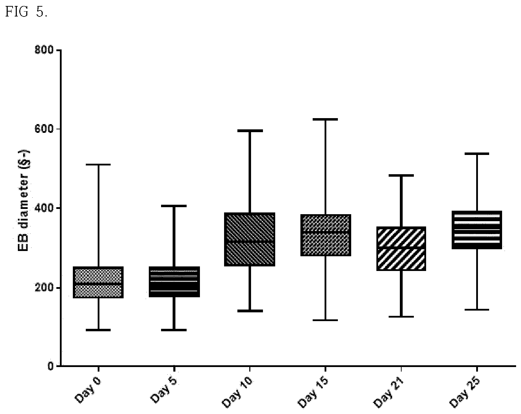

Regarding the process in which the diameter changes in each step, as shown in FIG. 5, it can be confirmed that human pluripotent stem cell-derived embryonic bodies having a diameter of 200 um to 250 µm finally become matured cardiac organoids having a diameter of 300 um to 400 µm on day 25.

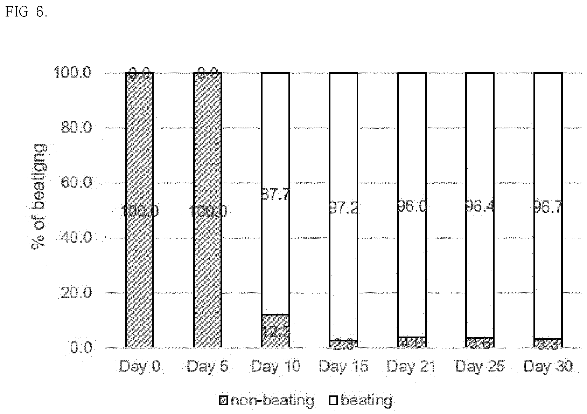

In addition, regarding the process in which the percentage of beating changes in each step, as shown in FIG. 6, it can be confirmed that, not only human pluripotent stem cell-derived embryonic bodies having a diameter of 200 um to 250 um on day 0, but also final matured cardiac organoids on days 21 to 30 show beating in 96% or more of subjects.

Above all, after step (S150) of maturing cardiac organoids is completed through the above-described sequential processes, cardiac organoids comprising self-organized cardiomyocytes, fibroblasts and endothelial cells at a cell number ratio of 55 to 65: 15 to 30: 15 through differentiation are obtained, and this cell number ratio is very close to a cell number ratio of 6: 2: 1.5, which is the number cell ratio between cardiomyocytes, fibroblasts and endothelial cells constituting an actual human heart.

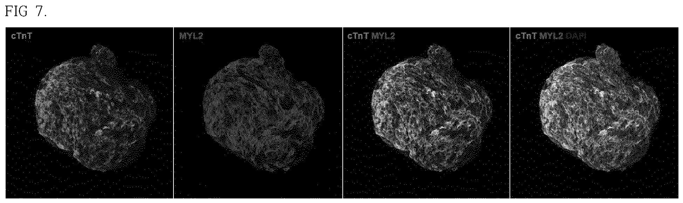

In this regard, fluorescence staining of human pluripotent stem cell-derived cardiac organoids was performed, and expression of the cardiomyocyte marker (cTnT) and the ventricular cardiomyocyte marker (MYL2) was examined by analysis of the fluorescence staining images. The results are shown in FIG. 7.

The cell number ratio between cardiomyocytes, fibroblasts and endothelial cells in the cardiac organoids is similar or identical to that in an actual heart, and the cardiac organoids obtained through simultaneous differentiation, self-organization of cells by interaction from the initial stage of development, and maturation are sufficient to mimic the structure and function of an actual heart, and thus the use thereof in drug screening and cardiac toxicity evaluation through disease modeling during new drug development may dramatically increase the efficiency of new drug development.

### 2. Description of experiment for confirmation of function of human pluripotent stem cell-derived cardiac organoids produced by method for producing human pluripotent stem cell-derived cardiac organoids

First, human pluripotent stem cell-derived cardiac organoids produced through the above-described method for producing human pluripotent stem cell-derived multi-cellular cardiac organoids were as one experimental group, and for comparison with this experimental group, another experimental group was set. The results of comparing the suitability of these experimental groups for an actual heart in terms of structure and function will be described below.

### (1) Preparation of experimental group of human pluripotent stem cell-derived cardiac organoids

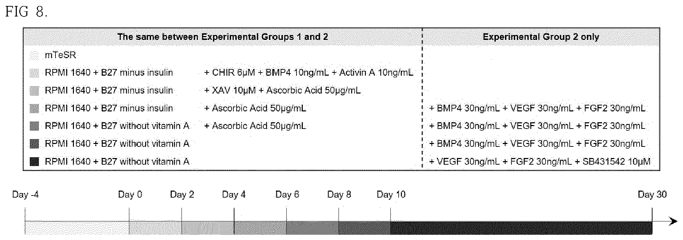

As shown in FIG. 8, human pluripotent stem cell-derived cardiac organoids obtained through differentiation under different culture conditions were set as Experimental Group 1 and Experimental Group 2, respectively.

For Experimental Group 1 and Experimental Group 2, the process of preparing human pluripotent stem cell-derived embryonic bodies through embryonic body preparation step (S110) and embryonic body culturing step (S120) were the same between the experimental groups.

In addition, human pluripotent stem cell-derived embryonic bodies with a diameter of 200 µm to 250 µm are primarily cultured in RPMI 1640 medium containing B-27 supplement (minus insulin), 6 µM of CHIR99021, 10 ng/ml of BMP4 and Activin A for 2 days. Then, further culturing the cultured human pluripotent stem cell-derived embryonic bodies for 2 days in an RPMI 1640 medium supplemented with B-27 supplement (minus insulin), 10 µM of XAV939 and 50 µg/ml of L-ascorbic acid, thus differentiating the embryonic bodies into structures comprising mesoderm cells and endoderm cells, was also the same between the experimental groups. Subsequent processes were performed under different culture conditions.

### - Experimental Example 1

For Experimental Group 1, as shown in FIG. 8, a process of inducing differentiation into cardiomyocytes by culturing structures comprising differentiated mesoderm cells and endoderm cells for 2 days in an RPMI 1640 medium supplemented with B-27 supplement (minus insulin) and 50 µg/ml of L-ascorbic acid was performed, and then the structures were cultured for 2 days in an RPMI 1640 medium supplemented with B-27 supplement (minus vitamin A) and 50 µg/ml of L-ascorbic acid, and then cultured for 2 days in an RPMI 1640 supplemented with B-27 supplement (minus vitamin A) without L-ascorbic acid, thus obtaining cardiac organoids through self-organization.

Next, the self-organized cardiac organoids corresponding to Experimental Group 1 were cultured and matured for 10 to 30 days in an RPMI 1640 medium supplemented with B-27 supplement (minus vitamin A), thus obtaining Experimental Group 1.

### - Experimental Group 2

For Experimental Group 2, as shown in FIG. 8, a process of inducing differentiation into cardiomyocytes by culturing structures comprising differentiated mesoderm cells and endoderm cells for 2 days in an RPMI 1640 medium supplemented with B-27 supplement (minus insulin) and 50 µg/ml of L-ascorbic acid was performed, and then the structures were cultured for 2 days in the above process and further supplemented with 30 ng/ml of BMP4, 30 ng/ml of VEGF (vascular endothelial growth factor) and 30 ng/ml of FGF2 (fibroblast growth factor 2), and then cultured for 2 days in an RPMI 1640 medium supplemented with B-27 supplement (minus vitamin A), 30 µg/ml of L-ascorbic acid, 30 ng/ml of BMP4, 30 ng/ml of VEGF (vascular endothelial growth factor) and 30 ng/ml of FGF2 (fibroblast growth factor 2), and then cultured for 2 days in an RPMI 1640 medium supplemented with B-27 supplement (minus vitamin A), 30 ng/ml of BMP4, 30ng/ml of VEGF (vascular endothelial growth factor) and 30 ng/ml of FGF2 (fibroblast growth factor 2) without L-ascorbic acid, thus obtaining cardiac organoids through self-organization.

Next, the self-organized cardiac organoids corresponding to Experimental Group 2 were cultured and matured for 10 to 20 days in an RPMI 1640 medium supplemented with B-27 supplement (minus vitamin A), 30ng/ml of VEGF (vascular endothelial growth factor), 30 ng/ml of FGF2 (fibroblast growth factor 2) and 10 ng/ml of SB431542, thus obtaining Experimental Group 2.

### (2) Experiment for comparison of cardiac organoid cell composition between experimental groups

Comparative analysis of the cell compositions of the cardiac organoids corresponding to Experimental Group 1 and Experimental Group 2 prepared through the above-described methods was performed.

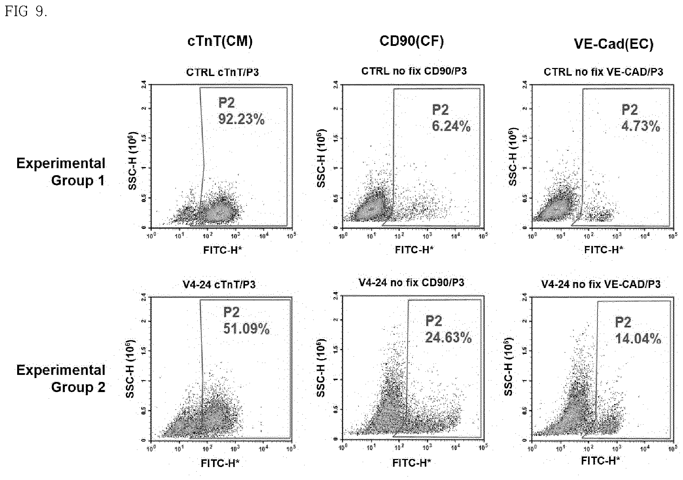

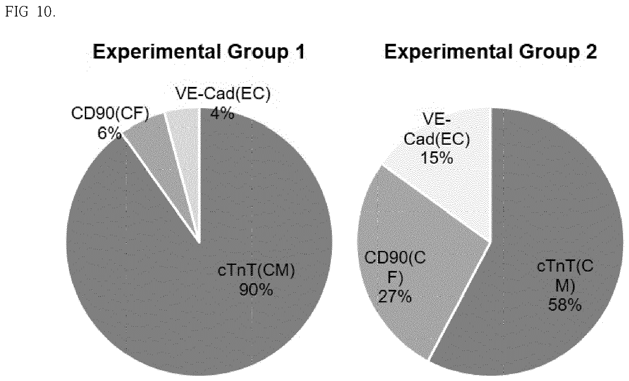

First, flow cytometry was performed using cTnT as a cardiomyocyte marker, CD90 as a fibroblast marker, and VE-Cadherin or CD31 as a vascular endothelial cell marker, and the results are shown in FIGS. 9 and 10.

In the case of Experimental Group 1, as shown in FIGS. 9 and 10, it was analyzed that the cardiomyocyte marker was 92%, the fibroblast marker was 6%, and the vascular endothelial cell marker was 4%, and in Experimental group 2, it was analyzed that the cardiomyocyte marker was 51%, the fibroblast marker was 24%, and the vascular endothelial cell marker was 14%.

More specifically, when each experimental result was converted into a percentage, Experimental Group 1 showed a composition ratio of 90% cardiomyocytes, 6% fibroblasts, and 4% endothelial cells, and Experimental Group 2 showed a composition ratio of 58% cardiomyocytes, 27% fibroblasts, and 15% endothelial cells.

Next, the relative expression levels of marker genes in the cardiac organoids corresponding to the experimental groups were analyzed through quantitative real-time PCR, and NKX2.5, TNNT2, MYL2 and MYL7 were used as cardiomyocyte markers.

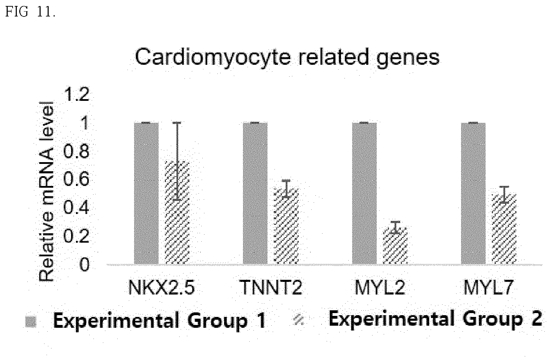

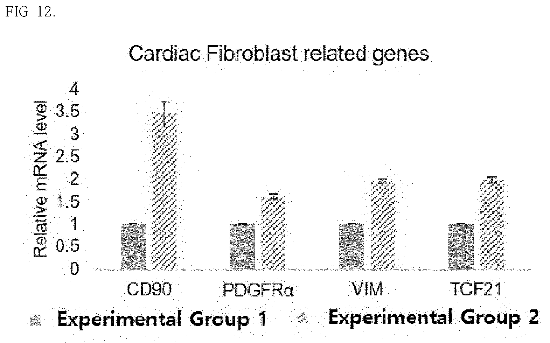

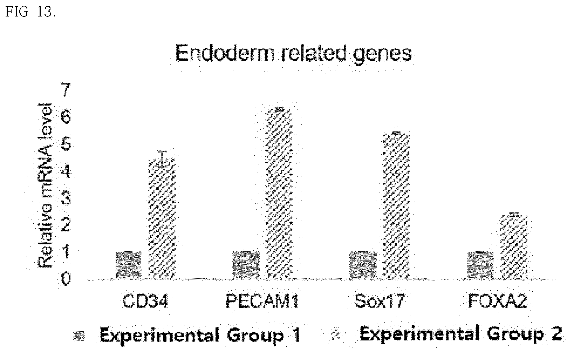

As a result, as shown in FIGS. 11 to 13, it can be confirmed that the expression levels were lower in Experimental Group 2 than in Experimental Group 1, and that the expression levels of the cardiac fibroblast markers CD90, PDGFR, Vimentin and TCF21, and the endoderm markers CD34, PECAM1, Sox17 and FOXA2 increased in the cardiac organoids of Experimental Group 2.

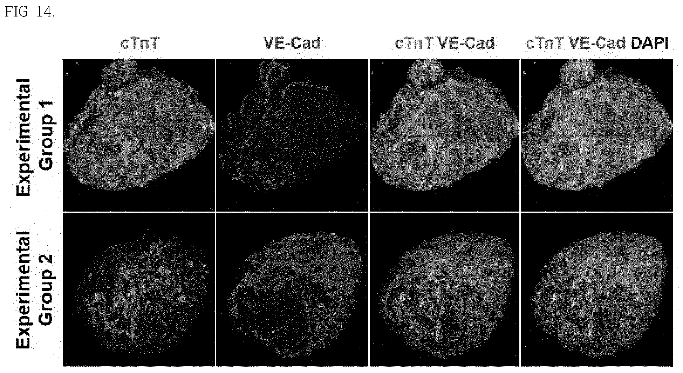

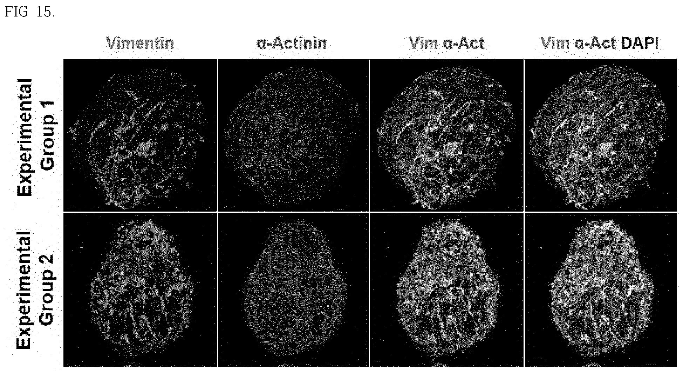

More specifically, as a result of performing immunofluorescence staining assay for the cardiac organoids of each of Experimental Group 1 and Experimental Group 2 prepared through the above-described methods, as shown in FIGS. 14 and 15, it can be seen that, in the cardiac organoids of Experimental Group 2, the distribution of the vascular endothelial cell marker (VE-Cadherin) and the fibroblast marker (Vimentin) increased, and expression of the cardiomyocyte marker (cTnT) generally decreased.

In particular, it can be seen that, in the cardiac organoids of Experimental Group 2, the vascular endothelial cell marker VE-Cadherin is distributed in a form surrounding the outermost of the cardiac organoids, compared to Experimental Group 1.

### (3) Experiment for comparison of calcium ion channel function of cardiac organoids between experimental groups

The calcium ion channel function of the cardiac organoids was measured and compared between Experimental Group 1 and Experimental Group 2.

First, to measure the concentration of calcium in living cells, changes in the calcium ion concentration of the cardiac organoids of each experimental group were observed using cell-permeable Fluo-4 AM (Thermo Fisher Scientific, MA, USA).

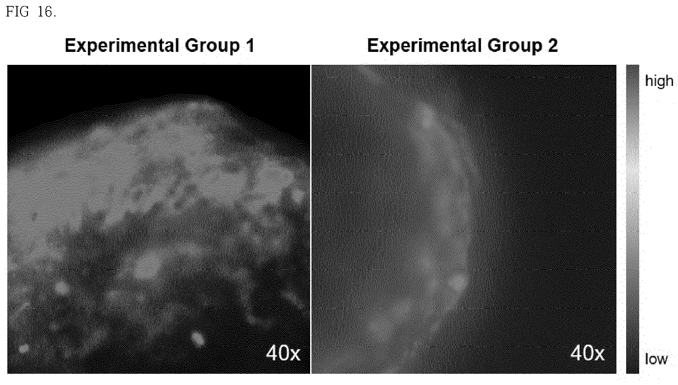

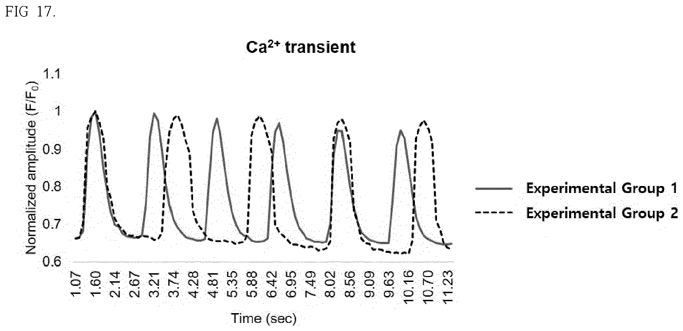

As a result, as shown in FIGS. 16 and 17, it could be confirmed that calcium ion movement and amplitude were relatively slower in Experimental Group 2, obtained by simultaneous differentiation into cardiomyocytes, fibroblasts and endothelial cells, than in Experimental Group 1 comprising more than 92% cardiomyocytes.

In summary, the prepared cardiac organoids of Experimental Group 2 overcome the limitations of lack of cardiac stem cells, are obtained by culture and differentiation, including simultaneous differentiation into cardiomyocytes, cardiac fibroblasts and vascular endothelial cells, which constitute the heart, from human pluripotent stem cells, and the self-organization of cells through interaction from the initial stage of development, and are sufficient to mimic the structure and function of an actual heart.

Accordingly, Experimental Group 2, which corresponds to a suitable example of human pluripotent stem cell-derived cardiac organoids produced through the above-described method for producing human pluripotent stem cell-derived cardiac organoids, is sufficient to mimic the structure and function of the heart, may dramatically increase the efficiency of new drug development through the use thereof in drug screening and cardiac toxicity evaluation through disease modeling during new drug development, and is suitable for use an alternative model in studies on heart-related disease mechanisms.

The embodiments disclosed in the present invention are not intended to limit the technical spirit of the present invention, but are intended to describe the invention, and the scope of the technical spirit of the present invention is not limited by these embodiments. The scope of protection should be construed by the appended claims, and all technical ideas within the scope equivalent thereto should be construed as being falling within the scope of the present invention.

## Claims

1. A method for producing human pluripotent stem cell-derived cardiac organoids, the method comprising:
step (A) of preparing embryonic bodies having a diameter of 100 µm to 130 µm by culturing human pluripotent stem cells (hPSCs);
step (B) of culturing the embryonic bodies, prepared in step (A), until the embryonic bodies have a diameter of 200 um to 250 µm;
step (C) of differentiating the human pluripotent stem cell-derived embryonic bodies having a diameter of 200 um to 250 µm, obtained in step (B), into structures comprising mesoderm cells and endoderm cells;
step (D) of differentiating the structures comprising mesoderm cells and endoderm cells, obtained in step (C), into cardiac organoids comprising self-organized cardiomyocytes, fibroblasts and vascular endothelial cells; and
step of (E) culturing and maturing the cardiac organoids obtained by differentiation in step (D),
wherein the cardiac organoids matured in step (E) contain self-organized cardiomyocytes, fibroblasts and vascular endothelial cells at a cell number ratio of 55 to 65: 15 to 30: 15.

2. The method according to claim 1, wherein the human pluripotent stem cells in step (A) are at least one cell type selected from among human embryonic stem cells (hESCs) and human induced pluripotent stem cells (hiPSCs).

3. The method according to claim 2, wherein step (A) is a step of preparing the embryonic bodies having a diameter of 100 um to 130 um by plating human pluripotent stem cells in an mTeSR1 medium supplemented with a Rho-associated kinase (ROCK) inhibitor (Y-27632) and culturing the plated cells for 1 day.

4. The method according to claim 3, wherein step (B) is a step of preparing human pluripotent stem cell-derived embryonic bodies having a diameter of 200 um to 250 um by culturing the embryonic bodies having a diameter of 100 um to 130 µm, prepared in step (A), in mTeSR medium free of a Rho-associated kinase (ROCK) inhibitor (Y-27632) for 2 to 3 days.

5. The method according to claim 1, wherein step (C) comprises:
step (C-1) of culturing the human pluripotent stem cell-derived embryonic bodies having a diameter of 200 um to 250 µm, prepared in step (B), for 2 days in an RPMI 1640 medium supplemented with B-27 supplement (minus insulin), CHIR99021, BMP4 (bone morphogenetic protein-4) and Activin A; and
step (C-2) of differentiating the human pluripotent stem cell-derived embryonic bodies, cultured in step (C-1), into structures comprising mesoderm cells and endoderm cells by culturing the embryonic bodies for 2 days in an RPMI 1640 medium supplemented with B-27 supplement (minus insulin), XAV939 and L-ascorbic acid.

6. The method according to claim 5, wherein step (C-1) is a step of culturing the human pluripotent stem cell-derived embryonic bodies having a diameter of 200 um to 250 um for 2 days in an RPMI 1640 supplemented with B-27 supplement (minus insulin), 6 µM to 8 µM of CHIR99021 as a GSK-3β (glycogen synthase kinase-3β) inhibitor for Wnt signal activation, 9 ng/ml to 10 ng/ml of BMP4 as a component for BMP signal activation, and 8 ng/ml to 10 ng/ml of Activin A as a component for TGF-β (transforming growth factor-β) signal activation

7. The method according to claim 6, wherein step (C-2) is a step of culturing the human pluripotent stem cell-derived embryonic bodies, cultured in step (C-1), for 2 days in an RPMI 1640 medium supplemented with B-27 supplement (minus insulin), 8 µM to 12 µM of XAV939 as a component for Wnt signaling inhibition and 40 ug/ml to 60 µg/ml of L-ascorbic acid as a component for BMP signal activation.

8. The method according to claim 5, wherein step (D) comprises:
step (D-1) of culturing the structures comprising mesoderm cells and endoderm cells, obtained by differentiation in step (C), for 2 days in a RPMI 1640 medium supplemented with B-27 supplement (minus insulin) and 40 µg/ml to 60 µg/ml of L-ascorbic acid, thereby inducing differentiation into cardiomyocytes;
step (D-2) of culturing the structures, whose differentiation into cardiomyocytes has been induced in step (D-1), for 2 days in the RPMI 1640 medium used in step (D-1) and further supplemented with 20 ng/ml to 40 ng/ml of BMP4, 20 ng/ml to 40 ng/ml of VEGF (vascular endothelial growth factor) and 20 ng/ml to 40 ng/ml of FGF2 (fibroblast growth factor 2), thereby inducing differentiation into fibroblasts and endothelial cells;
step (D-3) of culturing the structures, whose differentiation into cardiomyocytes, fibroblasts and vascular endothelial cells has been induced through step (D-2), for 2 days in an RPMI 1640 medium supplemented with B-27 supplement (minus vitamin A), 40 µg/ml to 60 µg/ml of L-ascorbic acid, 20 ng/ml to 40 ng/ml of BMP4, 20 ng/ml to 40 ng/ml of VEGF (vascular endothelial growth factor) and 20 ng/ml to 40 ng/ml of FGF2 (fibroblast growth factor 2); and
step (D-4) of obtaining cardiac organoids through differentiation and self-organization by culturing the structures, cultured in step (D-3), for 2 days in an RPMI 1640 medium supplemented with B-27 supplement (minus vitamin A), 20 ng/ml to 40 ng/ml of BMP4, 20 ng/ml to 40 ng/ml of VEGF (vascular endothelial growth factor) and 20 ng/ml to 40 ng/ml of FGF2 (fibroblast growth factor 2).

9. The method according to claim 8, wherein step (E) is a step of maturing the cardiac organoids containing self-organized cardiomyocytes, fibroblasts and vascular endothelial cells, obtained by differentiation in step (D-4), by culturing the cardiac organoids for 10 to 20 days in an RPMI 1640 medium supplemented with B-27 supplement (minus vitamin A), 20 ng/ml to 40 ng/ml of VEGF (vascular endothelial growth factor), 20 ng/ml to 40 ng/ml of FGF2 (fibroblast growth factor 2), and 10 ng/ml to 15 ng/ml of SB431542 as a component for TGF-β (transforming growth factor-β) signal inhibition.

10. Human pluripotent stem cell-derived cardiac organoids produced by the method for producing human pluripotent stem cell-derived cardiac organoids according to any one of claims 1 to 9.
